# EUROPEAN PATENT SPECIFICATION

(11) **EP 1 682 679 B1**
(45) Date of publication and mention of the grant of the patent: **02.04.2014**
(21) Application number: 04798436.4
(22) Date of filing: 09.11.2004
(51) Int. Cl.: C12Q 1/68

(54) **MOLECULAR MARKER**
MOLEKULARER MARKER
MARQUEUR MOLECULAIRE

(30) Priority: 10.11.2003 GB 0326197
(43) Date of publication of application: 26.07.2006
(73) Proprietor: Randox Laboratories Ltd., Crumlin, County Antrim BT29 4QY (GB)
(72) Inventor: CROCKARD, Martin Andrew, Randox Laboratories Ltd., Crumlin, Co. Antrim BT29 4QY (GB); BAILIE, Janice Roberta, Crossgar, County Down, BT30 9NW (GB); LAMONT, John Victor, Randox Laboratoires Ltd., Crumlin, Co. Antrim BT29 4QY (GB); FITZGERALD, Stephen Peter Randox Laboratoires Ltd., Crumlin, Co. Antrim BT29 4QY (IE)
(74) Representative: Jappy, John William Graham
(86) International application number: PCT/GB2004/004713
(87) International publication number: WO 2005/047539

(56) References cited:
- WO-A-02/055988
- US-A1- 2003 144 232
- DATABASE Geneseq [Online] 18 December 2003 (2003-12-18), "Human novel cDNA sequence, SEQ ID NO:595." XP002316183 retrieved from EBI accession no. GSN:ADC30513 Database accession no. ADC30513 -& WO 03/029271 A2 (HYSEQ, INC; TANG, TOM, Y; ZHANG, JIE; REN, FEIYAN; XUE, AIDONG, J; ZHA) 10 April 2003 (2003-04-10)
- DATABASE Geneseq [Online] 18 December 2003 (2003-12-18), "Human novel polypeptide sequence, SEQ ID NO:1566." XP002316184 retrieved from EBI accession no. GSP:ADC31484 Database accession no. ADC31484 -& WO 03/029271 A2 (HYSEQ, INC; TANG, TOM, Y; ZHANG, JIE; REN, FEIYAN; XUE, AIDONG, J; ZHA) 10 April 2003 (2003-04-10)
- DATABASE EMBL [Online] 3 December 1999 (1999-12-03), "Homo sapiens genomic DNA, chromosome 11 clone:RP11-876F8, complete sequence." XP002316185 retrieved from EBI accession no. EM_HUM:AP000795 Database accession no. AP000795

## Description

### Field of the Invention

This invention relates to the detection of the presence of or the risk of cancer, in particular breast cancer.

### Background of the Invention

There are over 1 million cases of breast cancer per year on a global basis, of which around 0.5 million are in the US, 40,000 are in the UK and nearly 2,000 in Ireland. It is the leading cause of cancer deaths among women. Although the overall incidence of the disease is increasing within the western world, wider screening and improved treatments have led to a gradual decline in the fatality rate of about 1% per year since 1991. Patients diagnosed with early breast cancer have greater than a 90% 5 year relative survival rate, as compared to 20% for patients diagnosed with distally metastasised breast cancer. Nonetheless, there is no definitive early-stage screening test for breast cancer, diagnosis currently being made on the results of mammography and fine needle biopsy. Mammography has its limitations, with over 80% of suspicious results being false positives and 10-15% of women with breast cancer providing false negative results. Often the tumour has reached a late stage in development before detection, reducing the chances of survival for the patient and increasing the cost of treatment and management for the healthcare system. More sensitive methods are required to detect small (<2 cm diameter) early stage *in-situ* carcinomas of the breast, to reduce patient mortality. In addition to early detection, there remain serious problems in classifying the disease as malignant or benign, in the staging of known cancers and in differentiating between tumour types. Finally, there is a need to monitor ongoing treatment effects and to identify patients becoming resistant to particular therapies. Such detection processes are further complicated, as the mammary gland is one of the few organs that undergo striking morphological and functional changes during adult life, particularly during pregnancy, lactation and involution, potentially leading to changes in the molecular signature of the same mammary gland over time.

Diagnosis of disease is often made by the careful examination of the relative levels of a small number of biological markers. Despite recent advances, the contribution of the current biomarkers to patient care and clinical outcome is limited. This is due to the low diagnostic sensitivity and disease specificity of the existing markers. Some molecular biomarkers, however, are being used routinely in disease diagnosis, for example prostate specific antigen in prostate cancer screening, and new candidate markers are being discovered at an increasing rate (Pritzker, 2002). It is becoming accepted that the use of a panel of well-validated biomarkers would enhance the positive predictive value of a test and minimize false positives or false negatives (Srinivas et al., 2002). In addition, there is now growing interest in neural networks, which show the promise of combining weak but independent information from various biomarkers to produce a prognostic/predictive index that is more informative than each biomarker alone (Yousef et al., 2002).

As more molecular information is collated, diseases such as breast cancer are being sub-divided according to genetic signatures linked to patient outcome, providing valuable information for the clinician. Emerging novel technologies in molecular medicine have already demonstrated their power in discriminating between disease sub-types that are not recognisable by traditional pathological criteria (Sorlie et al., 2001) and in identifying specific genetic events involved in cancer progression (Srinivas et al., 2002). Further issues need to be addressed in parallel, relating to the efficacy of biomarkers between genders and races, thus large scale screening of a diverse population is a necessity.

The management of breast cancer could be improved by the use of new markers normally expressed only in the breast but found elsewhere in the body, as a result of the disease. Predictors of the activity of the disease would also have valuable utility in the management of the disease, especially those that predict if a ductal carcinoma in situ will develop into invasive ductal carcinoma.

### Summary of the Invention

According to a first aspect of the present invention, there is a method for the detection of the presence of or the risk of breast cancer in a patient, comprising the steps of:
detecting in an isolated sample the expression of the gene characterised by the nucleotide sequence identified as SEQ ID No. 1, or its complement, or a polynucleotide of at least 30 consecutive nucleotides that hybridises to the sequence (or its complement) under stringent hybridising conditions, wherein an increased expression of the gene compared to a normal, control sample indicates the presence of or the risk of breast cancer in the patient from whom the sample was isolated.

According to a second aspect of the invention, an isolated polynucleotide comprises the nucleotide sequence identified herein as SEQ ID No. 1, or its complement, or a polynucleotide of at least 30 consecutive nucleotides that hybridises to the sequence (or its complement) under stringent hybridising conditions is used in an *in vitro* diagnostic assay to test for the risk of breast cancer in a patient.

According to a third aspect of the invention, there is an isolated polynucleotide consisting of SEQ ID No.1.

### Description of the Drawings

The invention is described with reference to the accompanying figures, wherein:
Figure 1 shows the results of a screening assay to determine the presence of the gene of interest in different tissues, T represents tumour tissue cDNA and M represents co-excised mammary tissue cDNA from the same donor;
Figure 2 shows the results of an expression analysis carried out to determine the expression of the gene of interest in different tissue samples; and Figure 3 shows the results of semi-quantitative PCR expression analysis of the gene of interest against a panel of 30 human tissue cDNA samples. Description of the Invention

The present invention is based on the identification of a gene that is expressed in a patient suffering breast cancer. Identification of the gene (or its expressed product) in a sample obtained from a patient indicates the presence of or the risk of breast cancer in the patient.

The disclosure further relates to reagents such as polypeptide sequences, useful for detecting, diagnosing, monitoring, prognosticating, preventing, imaging, treating or determining a pre-disposition to cancer.

The methods to carry out the diagnosis can involve the synthesis of cDNA from mRNA in a test sample, amplifying as appropriate portions of the cDNA corresponding to the gene or a fragment thereof and detecting the product as an indication of the presence of the disease in that tissue, or detecting translation products of the mRNAs comprising gene sequences as an indication of the presence of the disease.

Useful reagents include polypeptides or fragment(s) thereof which may be useful in diagnostic methods such as RT-PCR, PCR or hybridisation assays of mRNA extracted from biopsied tissue, blood or other test samples; or proteins which are the translation products of such mRNAs; or antibodies directed against these proteins. These assays also include methods for detecting the gene products (proteins) in light of possible post-translational modifications that can occur in the body, including interactions with molecules such as co-factors, inhibitors, activators and other proteins in the formation of sub-unit complexes.

The gene associated with cancer, is characterised by the polynucleotide shown as SEQ ID No. 1. The putative coding sequence is shown as SEQ ID No. 2. The expressed product of the gene is identified herein by SEQ ID No. 3. Identification of the gene or its expressed product may be carried out using techniques known for the detection or characterisation of polynucleotides or polypeptides. For example, isolated genetic material from a patient can be probed using short oligonucleotides that hybridise specifically to the target gene. The oligonucleotide probes may be detectably labelled, for example with a fluorophore, so that, upon hybridisation with the target gene, the probes can be detected. Alternatively, the gene, or parts thereof, may be amplified using the polymerase chain reaction, with the products being identified, again using labelled oligonucleotides.

Diagnostic assays incorporating this gene, or associated protein or antibodies will include, but are not limited to:
Polymerase chain reaction (PCR)
Reverse transcription PCR
Real-time PCR
In-Situ hybridisation
Southern dot blots
Immuno-histochemistry
Ribonuclease protection assay
cDNA array techniques
ELISA
Protein, antigen or antibody arrays on solid supports such as glass or ceramics, useful in binding studies.
Small interfering RNA functional assays.
All of the above techniques are well known to those in the art.

The present disclosure is concerned with the detection of isolated polynucleotides that comprise the sequence identified as SEQ ID No. 1 or SEQ ID No. 2, or its complement, or fragments thereof that comprise at least 15 consecutive nucleotides, preferably 30 nucleotides, more preferably at least 50 nucleotides. Hybridisation will usually be carried out under stringent conditions. Stringent hybridising conditions are known to the skilled person, and are chosen to reduce the possibility of non-complementary hybridisation. Examples of suitable conditions are disclosed in Nucleic Acid Hybridisation. A Practical Approach (B.D. Hames and S.J. Higgins, editors IRL Press, 1985). More specifically, stringent hybridisation conditions include overnight incubation at 42°C in a solution comprising: 50% formamide, 5 x SSC (150 mM NaCl, 15 mM trisodium citrate), 50 mM sodium phosphate (ph7.6), 5 x Denhardt's solution, 10% dextran sulphate and 20 µg/ml denatured, sheared salmon sperm DNA, followed by washing in 0.1 x SSC at about 65°C.

The identification of the gene also permits therapies to be developed, with the gene being a target for therapeutic molecules. For example, there are now many known molecules which have been developed for gene therapy, to target and prevent the expression of a specific gene. One particular molecule is a small interfering RNA (siRNA), which suppresses the expression of a specific target protein by stimulating the degradation of the target mRNA. Other synthetic oligonucleotides are also known which can bind to a gene of interest (or its regulatory elements) to modify expression. Peptide nucleic acids (PNAs) in association with DNA (PNA-DNA chimeras) have also been shown to exhibit. strong decoy activity, to alter the expression of the gene of interest. These molecules may be used to bind to the gene or its regulatory upstream elements, preventing expression.

The present disclosure also relates to the detection of isolated polypeptide products of the gene of interest. The polypeptide may be useful in the generation of antibodies or in the development of protein binding molecules that can bind in *vivo* to the protein to inhibit its activity.

Antibodies raised against a peptide of the invention will usually have an affinity for the peptide of at least 10⁻⁶M, more preferably, 10⁻⁹M and most preferably at least 10⁻¹¹M. The antibody may be of any suitable type, including monoclonal or polyclonal.

Detection of antibodies that specifically bind to the antigen in a test sample suspected of containing these antibodies may also be carried out. This detection method comprises contacting the test sample with a polypeptide which contains at least one epitope of the gene. Contacting is performed for a time and under conditions sufficient to allow antigen/antibody complexes to form. The method further entails detecting complexes, which contain the polypeptide. The polypeptide complex can be produced recombinantly or synthetically or be purified from natural sources.

In a separate embodiment of the invention, antibodies, or fragments thereof, against the antigen can be used for the detection of image localisation of the antigen in a patient for the purpose of detecting or diagnosing the disease or condition. Such antibodies can be monoclonal or polyclonal, or made by molecular biology techniques and can be labelled with a variety of detectable agents, including, but not limited to radioisotopes.

The term "antibody" refers broadly to any immunologic binding agent such as IgG, IgM, IgA, IgD and IgE. Antibody is also used to referto any antibody-like molecule that has an antigen-binding region and includes, but is not limited to, antibody fragments such as single domain antibodies (DABS), Fv, scFv, aptamers etc. The techniques for preparing and using various antibody-based constructs and fragments are well known in the art.

If desired, the cancer screening methods of the present invention may be readily combined with other methods in order to provide an even more reliable indication of diagnosis or prognosis, thus providing a multi-marker test.

The following example Illustrates the invention with reference to the accompanying drawings.

### Example

A number of differentially expressed gene fragments were isolated from cDNA populations derived from matched clinical samples of breast cancer patients, using non-isotopic differential display (DDRT-PCR). One of these fragments, referred to herein as DD20 was revealed to be significantly up-regulated in breast tumour tissue samples from a number of donors. The expression profile of this novel molecular marker, its full length and corresponding presumed protein sequence is detailed herein.

### Materials and methods

Differential gene expression between matched pairs of normal mammary and tumour tissue from the same donor was carried out. Tissue samples were obtained, with full ethical approval and informed patient consent, from Medical Solutions plc, Nottingham, UK. Following the surgical removal of a tumour, one sample of the tumour tissue was collected, as was a sample from the adjacent, co-excised normal tissue. Messenger RNA was extracted and cDNA subsequently synthesised, using Dynal dT₁₈-tagged Dynabeads and Superscript II reverse transcription protocols, respectively. Differential display reverse transcription PCR (DDRT-PCR) was employed to observe differences between the gene expression profiles of these matched samples, and individual gene transcripts showing up-or down-regulation were isolated and investigated further.

First described by Liang & Pardee (1992), differential display reverse transcription PCR (DDRT-PCR) uses mRNA from two or more biological samples as templates for representative cDNA synthesis by reverse transcription, with one of 3 possible anchor primers. Each of the 3 sub-populations was PCR-amplified using its respective anchor primer coupled with one of 80 arbitrary 13-mer primers. This number of primer combinations has been estimated to facilitate the representation of 96% of expressed genes in an mRNA population (Sturtevant, 2000). This population sub-division results in the reduction of the estimated 12,000-15,000 mRNAs expressed in eukaryotic cells to 100-150 transcripts by the end of second strand cDNA synthesis for each primer set. This facilitates the parallel electrophoretic separation and accurate visualization of matched primer sets on a polyacrylamide gel, leading to the identification of gene fragments expressed in one tissue sample but not the other.

Excision and re-amplification of fragments of interest was followed by removal of false positives through reverse Southern dot blotting. This entailed the spotting of each re-amplified fragment onto duplicate nylon membranes (Hybond N+, Amersham Pharmacia Biotech) and hybridising these with eitherthe tumour or normal tissue cDNA population of the donorfrom which the fragments were derived. Those fragments confirmed as differentially expressed were then direct-sequenced, i.e. without cloning, followed by web-based database interrogation to determine if each gene was novel. Fragments not matching know genes were regarded as potentially representing novel markers for the breast cancer from which they were derived. Further screening of each transcript was performed by either semi-quantitative RT-PCR or real-time PCR, using a suite of matched cDNA populations from a number of breast tumour donors. In all cases, β-actin was used as a constitutive reference gene, for calibrating the cDNA templates and as an internal positive control during PCR. Expression of each putative novel marker gene was performed through the use of gene-specific primer sets on the calibrated templates. Full-length transcripts of the novel gene fragments, including the open reading frame were then synthesized using 5' RACE (rapid amplification of cDNA ends), which incorporates gene-specific extension and amplification, verifiable by sequencing.

Determination of tissue specificity was assayed using the gene-specific primers from each novel marker against cDNA populations from non-breast tissue, including brain, heart, lymphocytes, spleen, kidney, testis and muscle (obtained from Origene). The DD20 molecular marker was further tested using cDNA populations derived from a more comprehensive panel of 22 human tissue types. These are as follows:

| | |
|---|---|
| Adrenal gland | pooled from 62 donors |
| Bone marrow | pooled from 7 donors |
| Brain, cerebellum | pooled from 24 donors |
| Brain, whole | pooled from 1 donor |
| Colon* | pooled from 1 donor |
| Foetal brain | pooled from 59 donors |
| Foetal liver | pooled from 63 donors |
| Heart | pooled from 1 donor |
| Kidney | pooled from 1 donor |
| Liver | pooled from 1 donor |
| Lung | pooled from 1 donor |
| Placenta | pooled from 7 donors |
| Prostate | pooled from 47 donors |
| Salivary gland | pooled from 24 donors |
| Skeletal muscle | pooled from 2 donors |
| Small intestine* | pooled from 1 donor |
| Spleen | pooled from 14 donors |
| Testis | pooled from 19 donors |
| Thymus | pooled from 9 donors |
| Thyroid gland | pooled from 65 donors |
| Trachea | pooled from 1 donor |
| Uterus | pooled from 10 donors |

Note that the majority of these samples were part of the Human Total RNA panel II (Clontech), but two samples, marked with asterisks, were obtained as tissue chunks from Medical Solutions plc. Nottingham, UK and processed at Randox Laboratories Ltd.

In addition, assays were performed on a range of ethically approved human tumour samples, as obtained through Medical Solutions plc. cDNA representative of tumours from ovary, testis, stomach, liver, lung, bladder, colon and pancreas were tested against both β-actin and DD20 by real-time PCR.

In conjunction with novel marker expression analysis, each matched pair of breast tissues was subjected to molecular signature analysis. This entailed using a suite of primers specific to a number of pre-published breast cancer molecular markers in semi-quantitative RT-PCR against each tissue cDNA. The relationship between each molecular marker was determined and tabulated for each sample and used as a reference, against which the novel markers could be compared. This was with the aim of sub-classifying the tumour types to enable the association of novel markers against such sub-types, increasing the power of the diagnostic marker considerably.

### Results and Discussion

Using differential display, a gene fragment, termed DD20, derived from cDNA populations of matched tissue from a breast cancer donor, was observed to have significant up-regulation in the tumour cDNA population in comparison to the corresponding normal tissue cDNA. This 187-nucleotide product was confirmed as differentially expressed by reverse Southern dot blots. Sequence analysis followed by database interrogation determined that DD20 was not homologous to known genes or proteins in the EMBL and SWISSPROT databases, respectively, so was regarded as potentially novel. It was, however, 100% homologous, after removal of the poly-A tail, to a clone from chromosome 11 of the human genome.

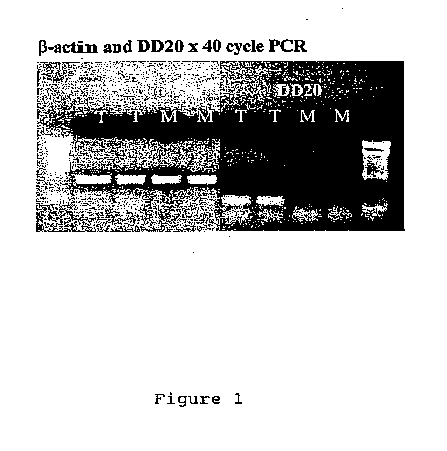

The tumour specificity of this fragment was confirmed, using gene specific primers, by semi-quantitative PCR against the originating donors matched tissue samples. These data suggest DD20 to be a putative marker for the presence of a breast tumour (Figure 1).

To facilitate further analysis, 5'-RACE was employed to extend the fragment to include the full open reading frame (ORF) of the gene, plus any 5' non-coding sequence. Using this technique, a presumed full-length product of 427 nucleotides was derived (SEQ. ID. No. 1), which on subsequent database interrogation, confirmed the previous homology to human chromosome 11, being 100% homologous over the full length of the sequence (427/427). From this sequence, all 6 amino acid reading frames were generated and a putative, small ORF was found in the +3 frame, comprising 67 amino acids, including the stop codon (SEQ ID No. 3). This small protein failed to reveal a high homology to any known proteins in the SWALL database, so is assumed to be novel. Initially, it was thought that this may be a small cytokine, as it shared a reasonable homology with the small inducible cytokine A22 precursors of both mouse and human, and was of a similar size to other cytokines in the SWISSPROT Database. However only one disulphide bridge (as indicated by the cysteine residues) is present in DD20; whereas all cytokines contain two disulphide bridges. Furthermore, this single bridge does not conform to either of those present in the cytokines.

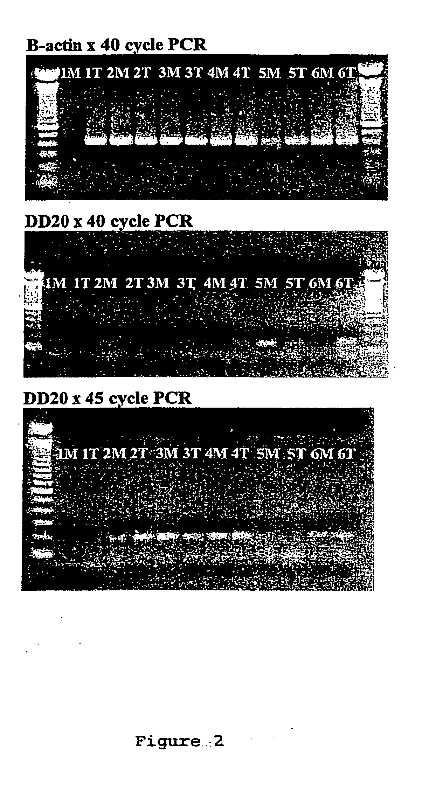

DD20 was further screened using semi-quantitative and real-time PCR analysis on cDNA populations derived from a number of matched breast tumour tissues donated by other patients. For conventional semi-quantitative PCR, 6 matched pairs of cDNA populations were assayed, initially at 40 cycles, then at 45 cycles of amplification due to the low levels of DD20 detected (Figure 2). β-actin was used for template calibration and as a positive control for PCR. In a number of these samples, notable increased expression was observed in the tumour samples, when compared to their normal counterparts. These data confirm DD20 to be a putative molecular marker for the presence of a breast tumour.

This analysis was substantiated by the molecular signature analysis of all currently available matched breast tissue samples, as follows;

| | | |
|---|---|---|
| Increased in tumour | 10 | 52.6% |
| Increased in normal | 3 | 15.8% |
| No discernable difference | 4 | 21.1% |
| No expression evident | 2 | 10.5% |
| Totals | 19 | 100% |

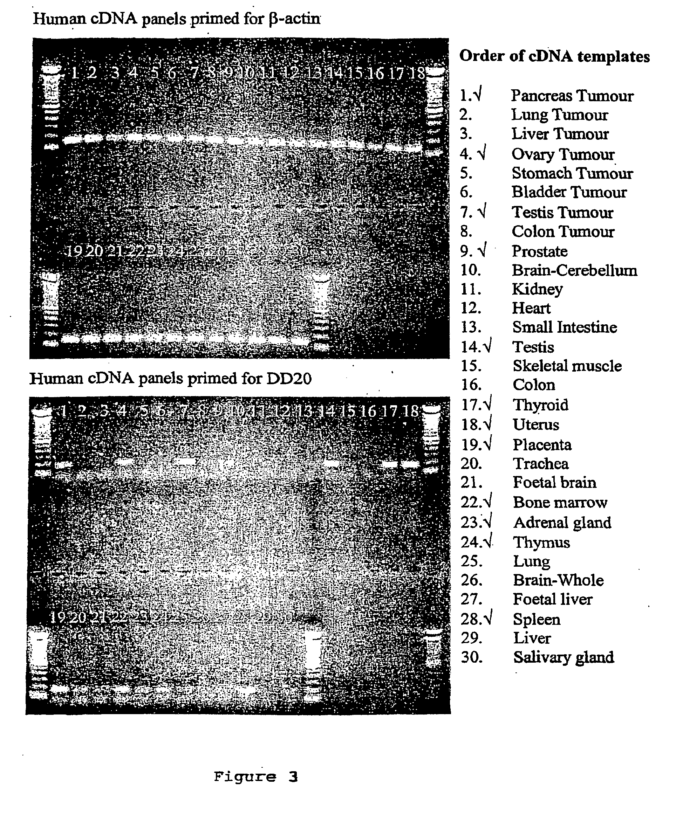

To determine organ specificity, cDNA populations from 22 non-breast human tissues were tested, both by conventional and real-time PCR, against the DD20 primers. In addition, 8 tumour tissue samples were analysed in the same way for DD20 expression. The same samples were also tested using primers from the constitutive housekeeping gene, β-actin, as a positive control and to calibrate the templates for semi-quantitative PCR analysis. The β-actin product was strongly amplified in all cDNA populations studied, confirming that the expression can be assumed to be semi-quantitative. Results of the conventional PCRs are given in Figure 3. From the panel of 30 tissue samples, DD20 appears to be selectively expressed. In most cases, strong expression of this putative marker is limited to tissues under the influence of reproductive hormones, for example ovary, testis, uterus and placenta. Weaker expression is also noted in other organs, such as bone marrow, spleen, thymus and thyroid. Of the tumours, expression is only strongly evident in the ovary and testis, and less so in the pancreas tumour.

Although not breast-specific or tumour-specific, this molecular marker shows significantly increased expression in a number of breast tumours and may relate to a specific sub-group or a tumour stage. As such, it could be useful for sub-classification of breast tumour type. Comparison of the expression profiles of DD20 in the tissue samples against the molecular signatures may reveal associations between this marker and other pre-published breast cancer markers, which have been linked to disease classification and prognosis.

For reference, it is important to point out that DD20 compares very favourably with some of the most highly regarded "standard" breast cancer markers, such as Oestrogen receptor (ERα) and human epidermal growth factor receptor (c-ErbB-2). This is evident both in the molecular signature analysis of all matched breast cancer tissue samples, where expression is similar in both samples from the same patient in many cases and using the target-specific primers against a panel of 30 cDNA populations from human normal and tumour tissue.

### References

DeRisi, J. L., lyer, V. R. and Brown, P. O. 1997. Exploring the metabolic and genetic control of gene expression on a genomic scale. Science. 278: 680-686.
Liang, P. and Pardee, A. B. 1992. Differential display of eukaryotic messenger RNA by means of the polymerase reaction. Science. 257: 967-971.
Pritzker, K. P. 2002 Cancer biomarkers: easier said than done. Clin. Chem. 2002 Aug; 48(8):1147-50.
Salodof MacNeil. 2001. From genes to proteins: The FLEXgene consortium. HMS Beagle. 112: on-line journal.
Sorlie T, Perou CM, Tibshirani R, Aas T, Geisler S, Johnsen H, Hastie T, Eisen MB, van de Rijn M, Jeffrey SS, Thorsen T, Quist H, Matese JC, Brown PO, Botstein D, Eystein Lonning P, Borresen-Dale AL. 2001. Gene expression patterns of breast carcinomas distinguish tumor subclasses with clinical implications. Proc Natl Acad Sci USA. Sep 11;98(19):10869-74.
Srinivas PR, Verma M, Zhao Y, Srivastava S. 2002. Proteomics for cancer biomarker discovery. Clin Chem. Aug;48(8):1160-9.
Sturtevant, J. Applications of differential-display reverse transcription-PCR to molecular pathogenesis and medical mycology. Clin Microbiol Rev. 2000 Jul;13(3):408-27.
Yousef et al., 2002 Yousef GM, Scorilas A, Kyriakopoulou LG, Rendl L, Diamandis M, Ponzone R, Biglia N, Giai M, Roagna R, Sismondi P, Diamandis EP. Human kallikrein gene 5 (KLK5) expression by quantitative PCR: an independent indicator of poor prognosis in breast cancer. Clin Chem. 2002 Aug;48(8):1241-50.
Zong, Q., Schummer, M., Hood, L. and Morris, D. R. 1999. Messenger RNA translation state: the second dimension of high-throughput expression screening. Proc. Natl. Acad. Sci. 96: 10632-10636

## Claims

1. A method for the detection of the presence of or the risk of breast cancer in a patient comprising the step of detecting in an isolated sample the expression of the gene **characterised by** the nucleotide sequence identified as SEQ ID No. 1, or its complement, or a polynucleotide of at least 30 consecutive nucleotides that hybridises to the sequence, or its complement, under stringent hybridising conditions, wherein an increased expression of the gene compared to a normal, control sample indicates the presence of or the risk of breast cancer in the patient from whom the sample was isolated.

2. A method according to claim 1, wherein the gene is that identified as SEQ ID No. 2.

3. A method according to claim 1 or claim 2, wherein the sample is obtained from breast tissue, the uterus, testis or ovary.

4. A method according to any preceding claim, wherein detection is carried out by amplifying the gene using the polymerase enzyme.

5. Use of a polynucleotide comprising the nucleotide sequence identified herein as SEQ ID No. 1, or its complement, or a polynucleotide of at least 30 consecutive nucleotides that hybridises to the sequence, or its complement, under stringent hybridising conditions in an *in vitro* diagnostic assay to test for the risk of breast cancer in a patient.

6. Use according to claim 6, wherein the sequence is that identified herein as SEQ ID No. 2.

7. An isolated polynucleotide consisting of SEQ ID No. 1.

## Patentansprüche

1. Verfahren zum Nachweis des Vorliegens von oder des Risikos für Brustkrebs bei einer Patientin, umfassend den Schritt des Nachweises der Expression des Gens in einer isolierten Probe, **gekennzeichnet durch** die als SEQ ID NO: 1 identifizierte Nukleotidsequenz, oder ihr Komplement, oder ein Polynukleotid aus mindestens 30 konsekutiven Nukleotiden, das unter stringenten Hybridisierungsbedingungen an die Sequenz, oder ihr Komplement, hybridisiert, wobei eine erhöhte Expression des Gens im Vergleich zu einer normalen Kontrollprobe das Vorliegen von oder des Risikos für Brustkrebs bei der Patientin anzeigt, aus dem die Probe isoliert wurde.

2. Verfahren nach Anspruch 1, wobei das Gen das als SEQ ID NO: 2 identifizierte ist.

3. Verfahren nach Anspruch 1 oder Anspruch 2, wobei die Probe aus dem Brustgewebe, dem Uterus, den Hoden oder dem Ovar gewonnen wird.

4. Verfahren nach einem vorangehenden Anspruch, wobei der Nachweis durch Amplifizieren des Gens unter Verwendung des Polymeraseenzyms durchgeführt wird.

5. Verwendung eines Polynukleotids, umfassend die hierin als SEQ ID NO: 1 identifizierte Nukleotidsequenz, oder ihr Komplement, oder ein Polynukleotid aus mindestens 30 konsekutiven Nukleotiden, das unter stringenten Hybridisierungsbedingungen in einem *in-vitro*-diagnostischen Assay an die Sequenz, oder ihr Komplement, hybridisiert, zum Testen auf das Risiko für Brustkrebs bei einer Patientin.

6. Verwendung nach Anspruch 6, wobei die Sequenz die hierin als SEQ ID NO: 2 identifizierte ist.

7. Isoliertes Polynukleotid bestehend aus SEQ ID NO: 1.

## Revendications

1. Méthode de détection de la présence de ou du risque de cancer du sein chez un(e) patient(e), comprenant l'étape qui consiste à détecter, dans un échantillon isolé, l'expression du gène **caractérisé par** la séquence nucléotidique identifiée comme étant la SEQ ID No. 1, ou son complément, ou un polynucléotide ayant au moins 30 nucléotides consécutifs qui hybride avec la séquence, ou son complément, sous des conditions d'hybridation stringentes, dans laquelle une expression accrue du gène, comparé à un échantillon témoin normal, indique la présence de ou le risque de cancer du sein chez le patient ou la patiente chez qui l'échantillon a été isolé.

2. Méthode selon la revendication 1, dans laquelle le gène est celui identifié comme étant la SEQ ID No. 2

3. Méthode selon la revendication 1 ou la revendication 2, dans laquelle l'échantillon provient de tissus mammaires, de l'utérus, des testicules ou de l'ovaire.

4. Méthode selon l'une quelconque des revendications précédentes, dans laquelle la détection est effectuée en amplifiant le gène et en utilisant l'enzyme polymérase.

5. Utilisation d'un polynucléotide comprenant la séquence de nucléotides identifiée ici comme étant la SEQ ID No. 1, ou son complément, ou un polynucléotide ayant au moins 30 nucléotides consécutifs qui hybride avec la séquence, ou son complément, sous des conditions d'hybridation stringentes dans le cadre d'un dosage diagnostique *in vitro* pour tester pour le risque de cancer du sein chez un patient ou une patiente.

6. Utilisation selon la revendication 6, dans laquelle la séquence est celle identifiée ici comme étant la SEQ ID No. 2.

7. Polynucléotide isolée constitué de la SEQ ID. No. 1.


### SEQUENCE LISTING

<110> Randox Laboratories Ltd.
<120> Molecular Marker
<130> JWJ01056WO
<150> 0326197.1
   <151> 2003-11-10
<160> 3
<170> PatentIn version 3.1
<210> 1
   <211> 427
   <212> DNA
   <213> Homo sapiens
<400> 1
   ctctccaaga gcttcaaact gagtaaccag caataatagt ctaccaactg ggaccaggac      60
   aaaggatggt aagagattct ctctgtggta gagaatggct gaaagcaggg gatggatcag     120
   caatactgaa aaaaacgttc tggtacccaa ggaaccactc taagcacaat gtacatattc     180
   tatcactgga ggaattggaa gtgtgtggta cacttcaggt aacaatagca aaaacaatta     240
   ccaaatctag tctaactact aactagattg actcaactca gaagtagagg tacacacatt     300
   tccaagagta aatactattt acttttgtat ctgctgtttt tccacataca attaccagta     360
   tttagtaaca attatgttct gtacccacaa aagcaagaaa gaatgacccc attgtcaaaa     420
   aaaaaaa                                                               427
<210> 2
   <211> 201
   <212> DNA
   <213> Homo sapiens
<220>
   <221> CDS
   <222> (1) .. (201)
   <223>
<400> 2
   atg gta aga gat tct ctc tgt ggt aga gaa tgg ctg aaa gca ggg gat       48
   gga tca gca ata ctg aaa aaa acg ttc tgg tac cca agg aac cac tct       96
   aag cac aat gta cat att cta tca ctg gag gaa ttg gaa gtg tgt ggt      144
   aca ctt cag gta aca ata gca aaa aca att acc aaa tct agt cta act      192
   act aac tag                                                          201
<210> 3
   <211> 66
   <212> PRT
   <213> Homo sapiens
<400> 3